# EUROPEAN PATENT APPLICATION

(11) **EP 4 233 974 A1**
(43) Date of publication of application: **30.08.2023**
(21) Application number: 21890930.7
(22) Date of filing: 21.10.2021
(51) Int. Cl.: A61M 25/10, A61M 29/02

(54) **BALLOON CATHETER**

(30) Priority: 13.11.2020 CN 202011272118
(71) Applicant: MicroPort NeuroTech (Shanghai) Co., Ltd., Shanghai 201318 (CN)
(72) Inventor: CUN, Yuxi, Shanghai 201318 (CN); LIU, Yunyun, Shanghai 201318 (CN); LIU, Yumei, Shanghai 201318 (CN); SUN, Li, Shanghai 201318 (CN)
(74) Representative: Marks & Clerk LLP
(86) International application number: PCT/CN2021/125445
(87) International publication number: WO 2022/100403

(57) **Abstract**

The present invention provides a balloon catheter including a tubular component and a balloon. The tubular component includes an inner tube and an outer tube. The balloon is secured to the tubular component and has an expanded configuration and a contracted configuration. The outer tube is sleeved over the inner tube. A first lumen is formed between the outer tube and the inner tube. The outer tube includes an outer tube main segment and a first recess. The first recess is located at a distal end of the outer tube main segment. An outer diameter of the first recess is smaller than an outer diameter of the outer tube main segment, and an inner diameter of the first recess is smaller than an inner diameter of the outer tube main segment. A proximal end of the balloon is fixedly connected to the first recess. The inner tube includes an inner tube main segment and a second recess. The second recess is located at a distal end of the inner tube main segment, and an outer diameter of the second recess is smaller than an outer diameter of the inner tube main segment. This arrangement imparts good accommodation performance to the balloon catheter and allows it to less rub or stimulate the wall of a blood vessel in which it is being advanced.

## Description

### TECHNICAL FIELD

The present invention relates to the field of medical devices and, in particular, to a balloon catheter.

### BACKGROUND

A balloon catheter typically includes a tubular component comprising a lumen and an expandable and collapsible balloon. In the interventional field, balloon catheters are used for expansion of stenosis and stents, occlusion of aneurysmal necks and occlusion of blood flow.

In some existing balloon catheter products, a balloon is usually arranged outside of an outer tube. In order to ensure smooth advancement of such a balloon catheter through a blood vessel, an outer diameter of the balloon catheter has to be limited. Moreover, as the balloon has a certain thickness itself, the balloon catheter's inner diameter tends to be too small to accommodate a relatively large medical instrument. In other instances, as a lumen for passage of a fluid tends to be too small, expansion or collapse of such a balloon catheter takes a very long time, undesirably prolonging a running time of a procedure using the balloon catheter. Further, arranging the balloon outside of the outer tube can stiffen a distal end of the balloon catheter, thus making it more strongly rub and stimulate the wall of a blood vessel and hard to push in which it is being advanced and degrading its bendability and accessibility.

These drawbacks may limit therapeutic efficacy of the balloon catheters, lead to increased surgical complexity and expose patients to greater risk.

### SUMMARY OF THE INVENTION

It is an object of the present invention to provide a balloon catheter, which overcomes the problems of poor accommodation performance, strong rubbing and stimulation of the wall of a blood vessel and poor pushability that may arise from the use of conventional balloon catheters.

To this end, the present invention provides a balloon catheter, comprising
a tubular component and a balloon, the tubular component comprising an inner tube and an outer tube, the balloon secured to the tubular component, the balloon having an expanded configuration and a contracted configuration, the outer tube sleeved over the inner tube, a first lumen formed between the outer tube and the inner tube.

The outer tube comprises an outer tube main segment and a first recess, the first recess located at a distal end of the outer tube main segment, the first recess having an outer diameter smaller than an outer diameter of the outer tube main segment, the first recess having an inner diameter smaller than an inner diameter of the outer tube main segment. A proximal end of the balloon is fixedly connected to the first recess.

The inner tube comprises an inner tube main segment and a second recess, the second recess located at a distal end of the inner tube main segment, the second recess having an outer diameter smaller than an outer diameter of the inner tube main segment.

Preferably, the first recess may comprise a first transition section and a first straight section from a proximal end to a distal end thereof, wherein the first transition section is a diameter-varying section where inner and outer diameters of the outer tube decrease.

Preferably, the first transition section may have an axial length of 0 mm to 10 mm.

Preferably, inner and outer surfaces of the first transition section may be inclined at a same angle of 0°-90° with respect to an axis of the outer tube main segment.

Preferably, a ratio of an outer diameter of the first straight section to the outer diameter of the outer tube main segment may be 0.7-1.0.

Preferably, the outer diameter of the outer tube main segment may be 1.0 mm to 3.7 mm, and the outer diameter of the first straight section may be 0.7 mm to 3.5 mm.

Preferably, the outer tube may further comprise an outer-tube distal section located at a distal end of the first recess, an outer diameter of the outer-tube distal section at a proximal end thereof is greater than the outer diameter of the first recess at the distal end thereof, the outer-tube distal section fixedly connected to the inner tube at a distal location.

Preferably, the outer-tube distal section may comprise a second transition section and a second straight section from a proximal end to a distal end thereof, wherein the second transition section is a diameter-varying section where an outer diameter of the outer tube increases.

Preferably, the second recess may comprise a third transition section and a third straight section from a proximal end to a distal end thereof, wherein the third transition section is a diameter-varying section where an outer diameter of the inner tube decreases.

Preferably, the second recess may have an axial length of 2-60 mm.

Preferably, an outer surface of the third transition section may be inclined at an angle of 0°-90° with respect to an axis of the inner tube main segment, and the third transition section may have an axial length of 0-10 mm.

Preferably, a ratio of an outer diameter of the third straight section to the outer diameter of the inner tube main segment may be greater than or equal to 0.6 and smaller than 1.0.

Preferably, the outer diameter of the inner tube main segment may be 0.5 mm to 3.2 mm, and the outer diameter of the third straight section may be greater than or equal to 0.3 mm and smaller than 3.2 mm.

Preferably, the inner tube may further comprise an inner-tube distal section located at a distal end of the second recess.

Preferably, the inner-tube distal section may have an axial length of 1-500 mm.

Preferably, an outer diameter of the inner-tube distal section may be smaller than the outer diameter of the second recess, and the inner-tube distal section may be located at a distal end of the balloon catheter.

Preferably, the inner-tube distal section may comprise a fourth transition section and a fourth straight section from a proximal end to a distal end thereof, wherein the fourth transition section is a diameter-varying section where an outer diameter of the inner tube decreases.

Preferably, the fourth straight section may have an outer diameter of 0.2 mm to 3.1 mm.

Preferably, a most distal end of the outer tube main segment may define a first transition location, and a most distal end of the inner tube main segment may define a second transition location, wherein the second transition location is located proximally with respect to the first transition location.

Preferably, the first recess may comprise a first transition section and a first straight section from a proximal end to a distal end thereof, and the second recess may comprise a third transition section and a third straight section from a proximal end to a distal end thereof,

wherein an outer surface of the third transition section forms a first angle with respect to an axis of the tubular component; an inner surface of the first transition section forms a second angle with respect to the axis of the tubular component; and the first angle is greater than or equal to the second angle.

Preferably, a projection of the second transition location on the axis of the tubular component may be 10 mm to 80 mm away from a projection of the first transition location on the axis of the tubular component.

Preferably, the projection of the second transition location on the axis of the tubular component may be 20 mm to 60 mm away from the projection of the first transition location on the axis of the tubular component.

Preferably, the projection of the second transition location on the axis of the tubular component may be 30 mm to 45 mm away from the projection of the first transition location on the axis of the tubular component.

Preferably, a distal end of the balloon may be fixedly connected to the second recess.

Preferably, the balloon may be disposed on the first recess, each of a proximal end and a distal end of the balloon fixedly connected to the outer tube, wherein a distal end of the outer tube is connected to the inner tube, and the first recess is provided with liquid passage apertures for dilation of the balloon with a liquid.

Preferably, the balloon may be made of any one of silicone, polyurethane, latex, polyethylene, polytrafluoroethylene and expanded polytrafluoroethylene, or of a mixture thereof.

Preferably, each of the inner and outer tubes may comprise at least one polymer layer made of one or more of polyether block amide, nylon, polyurethane, polytrafluoroethylene, polyethylene and polyolefin elastomer.

Preferably, the outer tube and/or the inner tube may further comprise reinforcing layer(s), the reinforcing layer is a structure braided from filaments, a structure consisting of spirally wound filaments, a cut tube or a combination thereof and is made of stainless steel, a nickel-titanium alloy, a cobalt-chromium alloy or a polymer.

Preferably, the outer tube and/or the inner tube may be triple-layered structure(s) each consisting of an innermost first polymer layer, an intermediate reinforcing layer and an outermost second polymer layer.

Preferably, the inner tube may provide therein with a second lumen having a constant inner diameter across its entire length.

Preferably, a ratio of the inner diameter of the second lumen to the outer diameter of the outer tube main segment may be 0.2-0.9.

Preferably, the inner diameter of the second lumen may be 0.1 mm to 3.0 mm, and the outer diameter of the outer tube main segment may be 0.5 mm to 3.7 mm.

Preferably, the balloon may have a length of 5-30 mm in the contracted configuration.

Preferably, the length of the balloon in the contracted configuration may be 10-20 mm.

In summary, the present invention provides a balloon catheter comprising a tubular component and a balloon, the tubular component comprising an inner tube and an outer tube, the balloon secured to the tubular component, the balloon having an expanded configuration and a contracted configuration, the outer tube sleeved over the inner tube, a first lumen is formed between the outer tube and the inner tube, the outer tube comprising an outer tube main segment and a first recess, the first recess located at a distal end of the outer tube main segment, the first recess having an outer diameter smaller than an outer diameter of the outer tube main segment, the first recess having an inner diameter smaller than an inner diameter of the outer tube main segment, a proximal end of the balloon fixedly connected to the first recess, the inner tube comprising an inner tube main segment and a second recess, the second recess located at a distal end of the inner tube main segment, the second recess having an outer diameter smaller than an outer diameter of the inner tube main segment.

The balloon catheter of the present invention offers at least one of the following benefits:
1. The recess located in the outer and inner tubes of the balloon catheter can accommodate at least a part of the balloon, allowing reduced joint thicknesses which can partially or completely eliminate the influence of the balloon on the balloon catheter's stiffness. In this way, it can be ensured that the balloon catheter has sufficient flexibility that allows its smooth advancement through a blood vessel.
2. The recess located in the outer and inner tubes of the balloon catheter enables the balloon catheter to have a reduced overall thickness, which allows the balloon catheter to have a sufficiently large inner cavity but not an excessive outer diameter. Thus, relatively large medical instruments can be delivered through the balloon catheter's inner cavity, and the catheter itself can be smoothly advanced within a tortuous blood vessel to access a relatively distal location in the blood vessel, while less stimulating the wall thereof.
3. A proximal end of the balloon is secured to the outer tube and a distal end thereof to the inner tube, additionally reducing the influence of the balloon on an overall outer diameter and compliance of the balloon catheter.
4. The recess located in the inner tube ensures a sufficient size of the lumen formed between the inner and outer tubes, and hence sufficiently fast expansion and collapse of the balloon.
5. The transition location of the outer tube where the outer diameter thereof starts varying is located distally with respect to the transition location of the inner tube where the outer diameter thereof starts varying. This ensures the size of the flow passage lumen will not shrink too much due to the reduced inner diameter of the outer tube, thus ensuring sufficient liquid passage or suction efficiency.
6. An axial distance between the transition location of the outer tube where the outer diameter thereof starts varying and the transition location of the inner tube where the outer diameter thereof starts varying is kept within a suitable range. In addition to a sufficient size of the flow passage lumen, this can further ensure good proximal supportability and distal flexibility of the balloon catheter, which enable the balloon catheter to have desirable ability to pass through bends and desirable accessibility.
7. The outer diameter of the inner tube at the distal end thereof is smaller than the outer diameter of the inner tube main segment at the proximal end thereof. This imparts to the catheter a compliance gradually increasing from the proximal to distal end, ensuring good delivery performance and desirable accessibility of the catheter.

### BRIEF DESCRIPTION OF THE DRAWINGS

Those of ordinary skill in the art would appreciate that the following drawings are presented merely to enable a better understanding of the present invention rather than limit the scope thereof in any sense. In the drawings,
Fig. 1 is a schematic overview of a balloon catheter according to a preferred embodiment of the present invention in an expanded configuration;
Fig. 2 is a schematic overview of a balloon catheter according to a preferred embodiment of the present invention in a contracted configuration;
Fig. 3 is a cross-sectional view of a distal portion of a balloon catheter according to a preferred embodiment of the present invention;
Fig. 4 is a cross-sectional view of a distal portion of a balloon catheter according to a preferred embodiment of the present invention;
Fig. 5 is a cross-sectional view of a distal portion of a balloon catheter according to a preferred embodiment of the present invention;
Fig. 6 is a cross-sectional view of a distal portion of a balloon catheter according to a preferred embodiment of the present invention;
Fig. 7 is a cross-sectional view of a distal portion of a balloon catheter according to a preferred embodiment of the present invention;
Fig. 8 is a schematic overview of a balloon catheter according to a preferred embodiment of the present invention;
Fig. 9 is a cross-sectional view of a distal portion of a balloon catheter according to a preferred embodiment of the present invention; and
Fig. 10 is a cross-sectional view of a distal portion of a balloon catheter according to a preferred embodiment of the present invention.

In these figures,
100, a tubular component; 200, a balloon; 101, an outer tube; 102, an inner tube; 1011, an outer tube main segment; 1012, a first recess; 1012-1, a first transition section; 1012-2, a first straight section; 1013, an outer-tube distal section; 1013-1, a second transition section; 1013-2, a second straight section; 1014, liquid passage apertures; 1021, an inner tube main segment; 1022, a second recess; 1022-1, a third transition section; 1022-2, a third straight section; 1023, an inner-tube distal section; 1023-1, a fourth transition section; 1023-2, a fourth straight section; 300, a first transition location; and 400, a second transition location.

### DETAILED DESCRIPTION

Objects, advantages and features of the present invention will become more apparent from the following more detailed description of particular embodiments made in conjunction with the accompanying drawings. Note that the figures are provided in a very simplified form not necessarily drawn to exact scale for the only purpose of helping to explain the disclosed embodiments in a more convenient and clearer way. In addition, structures shown in the figures are usually a part of actual structures. In particular, as the figures tend to have distinct emphases, they are often drawn to different scales.

As used herein and in the appended claims, the singular forms "a", "an" and "the" include plural referents unless the context clearly dictates otherwise. As used herein and in the appended claims, the term "or" is employed in the sense including "and/or" unless the context clearly dictates otherwise. Additionally, the terms "proximal" and "distal" are generally used to refer to an end closer to an operator and an end closer to a lesion site in a patient, respectively.

In principle, the present invention is intended to provide a balloon catheter including a tubular component and a balloon. The tubular component includes an inner tube and an outer tube. The balloon is secured to the tubular component and has an expanded configuration and a contracted configuration. The outer tube is sleeved over the inner tube so that a first lumen is formed between the outer tube and the inner tube. The outer tube includes an outer tube main segment and a first recess located at a distal end of the outer tube main segment. An outer diameter of the first recess is smaller than an outer diameter of the outer tube main segment. An inner diameter of the first recess is smaller than an inner diameter of the outer tube main segment. The balloon is fixedly connected at a proximal end thereof to the first recess. The inner tube includes an inner tube main segment and a second recess located at a distal end of the inner tube main segment. An outer diameter of the second recess is smaller than an outer diameter of the inner tube main segment.

A more detailed description is set forth with reference to the accompanying drawings.

### Embodiment 1

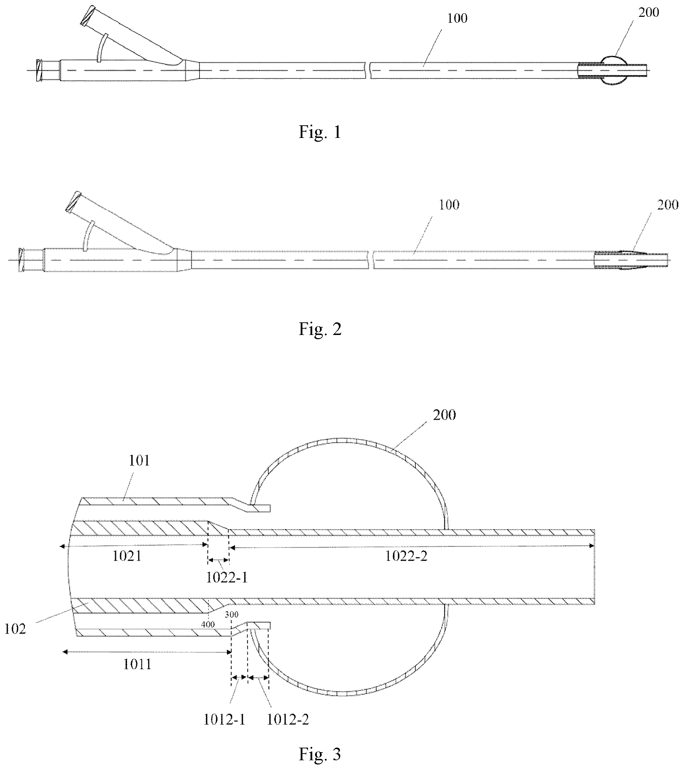

In a first embodiment of the present invention, there is provided a balloon catheter. Figs. 1 and 2 are schematic overviews of this balloon catheter, and Fig. 3 is a cross-sectional view of a distal portion of the balloon catheter. As shown in Figs. 1 to 3, the balloon catheter of the present invention includes a tubular component 100 and a balloon 200. The tubular component 100 includes an outer tube 101 and an inner tube 102, and the balloon 200 is secured to the tubular component 100. The outer tube 101 is sleeved over the inner tube 102 and a first lumen is formed between the outer tube 101 and the inner tube 102. The balloon 200 has an expanded configuration and a contracted configuration. Figs. 1 and 3 are a schematic overview of the balloon 200 in the expanded configuration and a cross-sectional view of a distal portion of the balloon, respectively. Fig. 2 is a schematic overview of the balloon 200 in the contracted configuration. The balloon 200 is able to switch between the expanded and contracted configurations. As shown in Fig. 3, the outer tube 101 includes an outer tube main segment 1011 and a first recess 1012 located at a distal end of the outer tube main segment 1011. A proximal end of the balloon 200 is secured to the first recess 1012 and a distal end of the balloon is secured to the inner tube 102. In this way, the balloon 200 is secured between the outer tube 101 and the inner tube 102. This can reduce influence of the presence of the balloon 200 on an overall outer diameter and compliance of the balloon catheter. The inner tube 102 of the balloon catheter includes an inner tube main segment 1021 and a second recess 1022 located at a distal end of the inner tube main segment 1021. An outer diameter of the second recess 1022 is smaller than an outer diameter of the inner tube main segment 1021. A proximal of the balloon 200 is secured to the first recess 1012 and a distal end thereof is secured to the second recess 1022. The recess arranged in the outer tube 101 and the inner tube 102 of the balloon catheter can accommodate at least a part of the balloon 200, allowing reduced joint thicknesses which can partially or completely eliminate the influence of the balloon on the balloon catheter's stiffness. In this way, it can be ensured that the balloon catheter has sufficient flexibility that allows its smooth advancement through a blood vessel. Moreover, the distal end of the balloon catheter is allowed to have increased flexibility, which additionally facilitates delivery of the balloon catheter through a blood vessel. Further, the recess arranged in the inner tube 102 ensures a passage of a large flow, avoiding slower expansion and contraction of the balloon due to a reduced size of the flow passage lumen.

In Embodiment 1, an outer diameter of the first recess 1012 is smaller than an outer diameter of the outer tube main segment 1011, and an inner diameter of the first recess 1012 is smaller than an inner diameter of the outer tube main segment 1011. A proximal end of the balloon 200 is secured to an outer surface of the first recess 1012.

In each case, an axial length of the first recess 1012 is 2-20 mm. In this embodiment, the axial length of the first recess 1012 is 12 mm. In some other embodiments, the axial length of the first recess 1012 may be 2 mm. In some other embodiments, the axial length of the first recess 1012 may be 5 mm. In some other embodiments, the axial length of the first recess 1012 may be 10 mm. In some other embodiments, the axial length of the first recess 1012 may be 15 mm. In some other embodiments, the axial length of the first recess 1012 may be 20 mm.

In each case, an axial length of the second recess 1022 is 2-60 mm. In Embodiment 1, the axial length of the second recess 1022 is 30 mm. In some other embodiments, the axial length of the second recess 1022 may be 2 mm. In some other embodiments, the axial length of the second recess 1022 may be 10 mm. In some other embodiments, the axial length of the second recess 1022 may be 25 mm. In some other embodiments, the axial length of the second recess 1022 may be 45 mm. In some other embodiments, the axial length of the second recess 1022 may be 60 mm.

As shown in Fig. 3, in Embodiment 1, the first recess 1012 includes a first transition section 1012-1 and a first straight section 1012-2 from the proximal end to the distal end thereof. An inner diameter of the first transition section 1012-1 varies from the inner diameter of the outer tube main segment 1011 to an inner diameter of the first straight section 1012-2, and an outer diameter of the first transition section 1012-1 varies from the outer diameter of the outer tube main segment 1011 to an outer diameter of the first straight section 1012-2. The first transition section 1012-1 is a diameter-varying section where both the inner and outer diameters of the outer tube 101 decrease.

As shown in Fig. 3, the second recess 1022 includes a third transition section 1022-1 and a third straight section 1022-2 from the proximal end to the distal end thereof. An outer diameter of the third transition section 1022-1 varies from the outer diameter of the inner tube main segment 1021 to an outer diameter of the third straight section 1022-2. The third transition section 1022-1 is a diameter-varying section where an outer diameter of the inner tube 102 decreases.

As shown in Fig. 3, in Embodiment 1, both inner and outer surfaces of the first transition section 1012-1 are inclined at a same angle of 45° with respect to an axis of the outer tube main segment 1011 (or of the tubular component 100). In some other embodiments, both the inner and outer surfaces of the first transition section 1012-1 are inclined at an angle with respect to the axis of the outer tube main segment 1011 (or of the tubular component 100), which may be any value greater than 0 and smaller than or equal to 90°. In some other embodiments, both the inner and outer surfaces of the first transition section 1012-1 may be inclined at a same angle of 60° with respect to the axis of the outer tube main segment 1011 (or of the tubular component 100). In some other embodiments, both the inner and outer surfaces of the first transition section 1012-1 may be inclined at a same angle of 5° with respect to the axis of the outer tube main segment 1011 (or of the tubular component 100). In some other embodiments, both the inner and outer surfaces of the first transition section 1012-1 may be inclined at a same angle of 85° with respect to the axis of the outer tube main segment 1011 (or of the tubular component 100). In some other embodiments, both the inner and outer surfaces of the first transition section 1012-1 may be perpendicular to the axis of the outer tube main segment 1011 (or of the tubular component 100). In each case, an axial length of the first transition section 1012-1 is 0-10 mm. In Embodiment 1, the axial length of the first transition section 1012-1 is 4 mm. In some other embodiments, the axial length of the first transition section 1012-1 may be 0 mm. In some other embodiments, the axial length of the first transition section 1012-1 may be 3 mm. In some other embodiments, the axial length of the first transition section 1012-1 may be 5 mm. In some other embodiments, the axial length of the first transition section 1012-1 may be 8 mm. In some other embodiments, the axial length of the first transition section 1012-1 may be 10 mm.

Similar to the first transition section 1012-1 of the first recess 1012, an outer surface of the third transition section 1022-1 is inclined at an angle of 10° with respect to an axis of the inner tube main segment 1021 (or of the tubular component 100). In some other embodiments, the outer surface of the third transition section 1022-1 may be inclined at an angle of 5° with respect to the axis of the inner tube main segment 1021 (or of the tubular component 100). In some other embodiments, the outer surface of the third transition section 1022-1 may be inclined at an angle of 15° with respect to the axis of the inner tube main segment 1021 (or of the tubular component 100). In some other embodiments, the outer surface of the third transition section 1022-1 may be inclined at an angle of 25° with respect to the axis of the inner tube main segment 1021 (or of the tubular component 100). In some other embodiments, the outer surface of the third transition section 1022-1 may be inclined at an angle of 20° with respect to the axis of the inner tube main segment 1021 (or of the tubular component 100). In some other embodiments, the outer surface of the third transition section 1022-1 may be perpendicular to the axis of the inner tube main segment 1021 (or of the tubular component 100). In some embodiments, an inner surface of the third transition section 1022-1 may be parallel to the axis of the inner tube main segment 1021 (or of the tubular component 100), and the outer surface of the third transition section 1022-1 may be inclined at an angle with respect to the axis of the inner tube main segment 1021 (or of the tubular component 100), which may be any value in the range of 0-90° such as 5°, 15°, 30°, 40°, 45°, 60°, 75° or 85°. In some other embodiments, the inner surface of the third transition section 1022-1 may be parallel to the axis of the inner tube main segment 1021 (or of the tubular component 100), and the outer surface of the third transition section 1022-1 may be perpendicular to the axis of the inner tube main segment 1021 (or of the tubular component 100). In some other embodiments, the inner surface of the third transition section 1022-1 may be inclined at an angle with respect to the axis of the inner tube main segment 1021 (or of the tubular component 100), which may be any value in the range of 0-90° such as 5°, 15°, 30°, 40°, 45°, 60°, 75° or 85°. In some other embodiments, the inner surface of the third transition section 1022-1 may be perpendicular to the axis of the inner tube main segment 1021 (or of the tubular component 100). In each case, an axial length of the third transition section 1022-1 is 0-10 mm. In Embodiment 1, the axial length of the third transition section 1022-1 is 5 mm. In some other embodiments, the axial length of the third transition section 1022-1 may be 0 mm. In some other embodiments, the axial length of the third transition section 1022-1 may be 3 mm. In some other embodiments, the axial length of the third transition section 1022-1 may be 5 mm. In some other embodiments, the axial length of the third transition section 1022-1 may be 8 mm. In some other embodiments, the axial length of the third transition section 1022-1 may be 10 mm.

In some embodiments, the outer diameter of the outer tube main segment 1011 may be 1.0 mm to 3.7 mm, the outer diameter of the first straight section 1012-2 may be 0.7 mm to 3.5 mm, and a ratio of the outer diameter of the first straight section 1012-2 to the outer diameter of the outer tube main segment 1011 is 0.7-1.0. In Embodiment 1, the outer diameter of the outer tube main segment 1011 is 2.8 mm, the outer diameter of the first straight section 1012-2 is 2.6 mm, and the ratio of the outer diameter of the first straight section 1012-2 to the outer diameter of the outer tube main segment 1011 is 0.928. In some other embodiments, the outer diameter of the outer tube main segment 1011 may be 3.7 mm, the outer diameter of the first straight section 1012-2 may be 2.8 mm, and the ratio of the outer diameter of the first straight section 1012-2 to the outer diameter of the outer tube main segment 1011 may be 0.757. In some other embodiments, the outer diameter of the outer tube main segment 1011 may be 3.5 mm, the outer diameter of the first straight section may be 3.5 mm, and the ratio of the outer diameter of the first straight section 1012-2 to the outer diameter of the outer tube main segment 1011 may be 1.0. In some other embodiments, the outer diameter of the outer tube main segment 1011 may be 1.0 mm, the outer diameter of the first straight section 1012-2 may be 0.7 mm, and the ratio of the outer diameter of the first straight section 1012-2 to the outer diameter of the outer tube main segment 1011 may be 0.7.

In some embodiments, the outer diameter of the inner tube main segment 1021 may be 0.5 mm to 3.2 mm, the outer diameter of the third straight section 1022-2 may be 0.3 mm to 3.2 mm, and a ratio of the outer diameter of the third straight section 1022-2 to the outer diameter of the outer tube main segment 1011 may be 0.6-1.0. In Embodiment 1, the outer diameter of the inner tube main segment 1021 is 2.8 mm, the outer diameter of the third straight section 1022-2 is 2.4 mm, and the ratio of the outer diameter of the third straight section 1022-2 to the outer diameter of the outer tube main segment 1011 is 0.857. In some other embodiments, the outer diameter of the inner tube main segment 1021 may be 3.2 mm, the outer diameter of the third straight section 1022-2 may be 3.2 mm, and a ratio of the outer diameter of the third straight section 1022-2 to the outer diameter of the inner tube main segment 1021 may be 1.0. In some other embodiments, the outer diameter of the inner tube main segment 1021 may be 0.5 mm, the outer diameter of the third straight section 1022-2 may be 0.3 mm, and the ratio of the outer diameter of the third straight section 1022-2 to the outer diameter of the inner tube main segment 1021 may be 0.6. In some other embodiments, the outer diameter of the inner tube main segment 1021 may be 1.0 mm, the outer diameter of the third straight section 1022-2 may be 0.8 mm, and the ratio of the outer diameter of the third straight section 1022-2 to the outer diameter of the inner tube main segment 1021 may be 0.8. In some other embodiments, the outer diameter of the inner tube main segment 1021 may be 2.0 mm, the outer diameter of the third straight section 1022-2 may be 1.8 mm, the ratio of the outer diameter of the third straight section 1022-2 to the outer diameter of the inner tube main segment 1021 may be 0.9.

As shown in Fig. 3, in the balloon catheter of Embodiment 1, the outer tube main segment 1011 comprises a first transition location 300 at its most distal end. In Embodiment 1, the outer and inner diameters of the outer tube 101 in the balloon catheter start varying at the first transition location 300. In some other embodiments, the outer diameter and/or the inner diameter of the outer tube 101 in the balloon catheter may start varying at the first transition location 300 (i.e., the first transition location is located at an interface between the outer tube main segment and the first recess). In the balloon catheter, the inner tube main segment 1021 comprises a second transition location 400 at its most distal end. In Embodiment 1, the outer diameter of the inner tube in the balloon catheter starts varying at the second transition location 400. In some other embodiments, both the outer and inner diameters of the inner tube 102 in the balloon catheter may start varying at the second transition location 400 (i.e., the second transition location is located at an interface between the inner tube main segment and the second recess). In the balloon catheter, the first transition location 300 may be a cross-section having the same cross-sectional shape as at the most distal end of the outer tube main segment 1011, and the second transition location 400 may be a cross-section having the same cross-sectional shape as at the most distal end of the inner tube main segment 1021. Hereinafter, for ease of description, they are referred to as the "first transition location 300" and the "second transition location 400", respectively. In Embodiment 1, a projection of the first transition location 300 on the axis of the tubular component 100 is located distally with respect to a projection of the second transition location 400 on the axis of the tubular component 100. Moreover, in Embodiment 1, the outer surface of the third transition section 1022-1 is inclined at a first angle with respect to the axis of the tubular component 100, and the inner surface of the first transition section 1012-1 is inclined at a second angle with respect to the axis of the tubular component 100. The first angle is greater than or equal to second angle. In Embodiment 1, the inner diameter of the outer tube 101 in the balloon catheter starts decreasing at the first transition location 300, and the outer diameter of the inner tube 102 in the balloon catheter starts decreasing at the second transition location 400. Through configuring the projection of the first transition location 300 on the axis of the tubular component 100 to be distal with respect to the projection of the second transition location 400 on the axis of the tubular component 100, and through configuring the angle between the third transition section 1022-1 and the axis of the tubular component 100 to be greater than the angle between the first transition section 1012-1 and the axis of the tubular component 100, it can be ensured that the size of the first lumen will not shrink too much due to the reduced inner diameter of the outer tube 101. When the first lumen is used to allow passage or suction of a liquid therethrough, this can ensure sufficient passage or suction efficiency.

In each case, the balloon catheter has an overall length of 80-160 cm. In Embodiment 1, the overall length of the balloon catheter is 130 cm. In some other embodiments, the overall length of the balloon catheter may be 80 cm. In some other embodiments, the overall length of the balloon catheter may be 160 cm. In some other embodiments, the overall length of the balloon catheter may be 115 cm. In some other embodiments, the overall length of the balloon catheter may be 110 cm. In some other embodiments, the overall length of the balloon catheter may be 140 cm. In some other embodiments, the overall length of the balloon catheter may be 150 cm.

In Embodiment 1, the balloon 200 is a polymer film. The first lumen is configured for passage or suction of a liquid therethrough, which can cause a switching of the balloon 200 between the expanded and contracted configurations. The liquid that can be passed or sucked through the first lumen may be, for example, a contrast fluid, a physiological saline solution or the like. Filling the first lumen with the liquid can bring the balloon 200 into the expanded configuration. Emptying the first lumen can bring the balloon 200 into the contracted configuration. In Embodiment 1, the polymer film has a thickness of 0.10 mm. In some other embodiments, the thickness of the polymer film may be 0.05 mm to 0.15 mm, such as 0.05 mm, 0.08 mm, 0.12 mm or 0.15 mm. In Embodiment 1, the polymer film is made of silicone. In some other embodiments, the polymer film may be made of polyurethane (PU). In some other embodiments, the polymer film may be made of latex. In some other embodiments, the polymer film may be made of polyethylene (PE). In some other embodiments, the polymer film may be made of polytrafluoroethylene (PTFE). In some other embodiments, the polymer film may be made of expanded PTFE (ePTFE). In some other embodiments, the polymer film may be made of PU and PE mixed at a ratio of 2: 1. In some other embodiments, the polymer film may be made of PTFE and ePTFE mixed at a ratio of 1: 1. In some other embodiments, the polymer film may be made of silicone, PU and PE mixed at a ratio of 1: 1: 1. In Embodiment 1, a proximal end of the balloon 200 is connected to the first straight section 1012-2, for example, by gluing, bonding or fusion. In some other embodiments, a proximal end of the balloon 200 may be connected to the first transition section 1012-1, for example, by gluing, bonding or fusion.

In Embodiment 1, the inner tube 102 is a triple-layered structure consisting of an innermost first polymer layer, an intermediate reinforcing layer and an outermost second polymer layer. The first polymer layer is made of PTFE, and the reinforcing layer is a structure braided from stainless steel filaments. The second polymer layer is composed of axially spliced polyether block amide (PEBA), nylon, PU, PE and polyolefin elastomer (POE). The outer tube 101 consists of a single polymer layer made of PEBA. In some other embodiments, both the inner tube 102 and the outer tube 101 may be triple-layered structures each consisting of an innermost first polymer layer, an intermediate reinforcing layer and an outermost second polymer layer. In some other embodiments, the inner tube 102 may be a single-layered polymer structure, and the outer tube 101 may be a triple-layered structure. In some other embodiments, the inner tube 102 may be a triple-layered structure, and the outer tube 101 may be a double-layered polymer structure. In some other embodiments, the reinforcing layer(s) in the inner tube 102 and/or the outer tube 101 may each be a structure consisting of spirally wound filaments. In some other embodiments, the reinforcing layer(s) in the inner tube 102 and/or the outer tube 101 may each be a cut tube. In some other embodiments, the reinforcing layer(s) in the inner tube 102 and/or the outer tube 101 may each consist of a structure braided from filament and a structure consisting of spirally wound filaments. In some other embodiments, the reinforcing layer(s) in the inner tube 102 and/or the outer tube 101 may each consisting of a structure braided from filament and a cut tube. In some other embodiments, the reinforcing layer(s) in the inner tube 102 and/or the outer tube 101 may each consist of a cut tube and a structure consisting of spirally wound filaments. In some other embodiments, the reinforcing layer(s) in the inner tube 102 and/or the outer tube 101 may each be made of a material including a nickel-titanium (NiTi) alloy. In some other embodiments, the reinforcing layer(s) in the inner tube 102 and/or the outer tube 101 may be each made of a material including a cobalt-chromium (CoCr) alloy. In some other embodiments, the reinforcing layer(s) in the inner tube 102 and/or the outer tube 101 may each be made of a material including a polymer. In some other embodiments, the reinforcing layer(s) in the inner tube 102 and/or the outer tube 101 may each be made of both a NiTi alloy and stainless steel. In some other embodiments, the reinforcing layer(s) in the inner tube 102 and/or the outer tube 101 may each be made of both a NiTi alloy and a polymer.

In Embodiment 1, the balloon catheter includes a first radiopaque ring disposed at an end of the balloon catheter and a second radiopaque ring disposed at a location of the inner tube 102 that is adapted to the location of the balloon 200.

In Embodiment 1, the inner tube 102 of the balloon catheter comprises a second lumen therein, which has a constant inner diameter across its entire length. In each case, the inner diameter of the second lumen is 0.1 mm to 3.0 mm, and the outer diameter of the outer tube main segment 1011 is 0.5 mm to 3.7 mm. In this embodiment, the inner diameter of the second lumen is 2.3 mm, the outer diameter of the outer tube main segment 1011 is 2.8 mm, and a ratio of the inner diameter of the second lumen to the outer diameter of the outer tube main segment 1011 is 0.821. In some other embodiments, the inner diameter of the second lumen may be 0.1 mm, the outer diameter of the outer tube main segment 1011 may be 0.5 mm, and the ratio of the inner diameter of the second lumen to the outer diameter of the outer tube main segment 1011 may be 0.2. In some other embodiments, the inner diameter of the second lumen may be 3.0 mm, the outer diameter of the outer tube main segment 1011 may be 3.6 mm, and the ratio of the inner diameter of the second lumen to the outer diameter of the outer tube main segment 1011 may be 0.833. In some other embodiments, the inner diameter of the second lumen may be 2.7 mm, the outer diameter of the outer tube main segment 1011 may be 3.0 mm, and the ratio of the inner diameter of the second lumen to the outer diameter of the outer tube main segment 1011 may be 0.9. In some other embodiments, the inner diameter of the second lumen may be 2.5 mm, the outer diameter of the outer tube main segment 1011 may be 3.7 mm, and the ratio of the inner diameter of the second lumen to the outer diameter of the outer tube main segment 1011 may be 0.676. In Embodiment 1, the second lumen is configured for passage of a medical instrument therethrough.

In Embodiment 1, a length of the balloon 200 in the contracted configuration is 10 mm. In other embodiments, the length of the balloon 200 in the contracted configuration may be 5-30 mm. In another embodiment, the length of the balloon 200 in the contracted configuration may be 5 mm. In another embodiment, the length of the balloon 200 in the contracted configuration may be 8 mm. In another embodiment, the length of the balloon 200 in the contracted configuration may be 15 mm. In another embodiment, the length of the balloon 200 in the contracted configuration may be 20 mm. In another embodiment, the length of the balloon 200 in the contracted configuration may be 24 mm. In another embodiment, the length of the balloon 200 in the contracted configuration may be 30 mm.

In Embodiment 1, there are angled transitions between the outer tube main segment 1011 and the first recess 1012 and between the first transition section 1012-1 and the first straight section 1012-2. In some other embodiments, there may be smooth curved transition(s) between the outer tube main segment 1011 and first recess 1012 and/or between the first transition section 1012-1 and the first straight section 1012-2. In Embodiment 1, the first straight section 1012-2 has a straight, smooth surface. In some other embodiments, the first straight section 1012-2 may provide on its surface with convexities and concavities, grooves or curved portions, while having constant inner and outer diameters across its entire length.

In Embodiment 1, there are angled transitions between the inner tube main segment 1021 and the second recess 1022 and between the third transition section 1022-1 and the third straight section 1022-2. In some other embodiments, there may be smooth curved transition(s) between the inner tube main segment 1021 and the second recess 1022 and/or between the third transition section 1022-1 and the third straight section 1022-2. In Embodiment 1, the third straight section 1022-2 has a straight, smooth surface. In some other embodiments, the third straight section 1022-2 may provide on its surface with convexities and concavities, grooves or curved portions, while having constant inner and outer diameters across its entire length.

### Embodiment 2

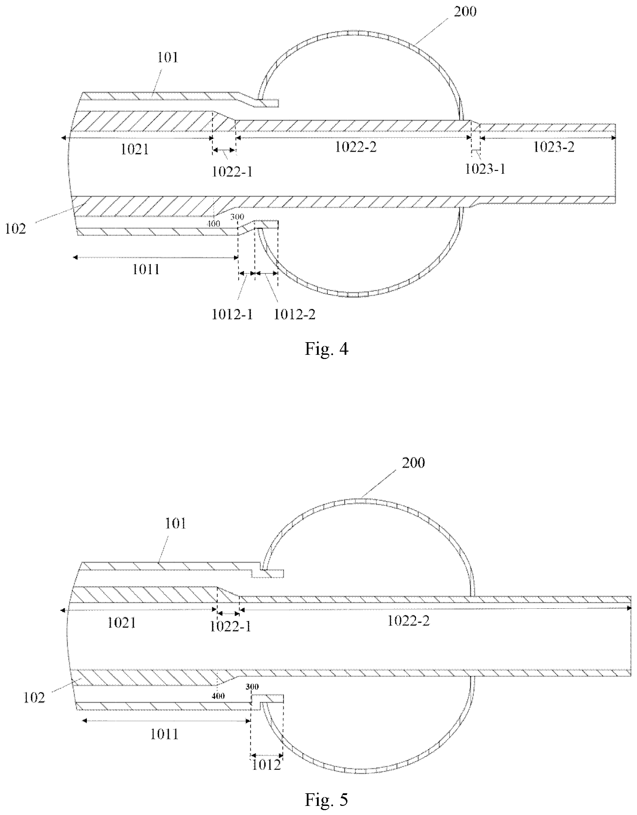

In a second embodiment of the present invention, there is provided a balloon catheter. Fig. 4 is a cross-sectional view of a distal portion of this balloon catheter with a balloon 200 thereof being in an expanded configuration. As shown in Fig. 4, the balloon catheter of Embodiment 2 has an overall structure substantially similar to that of the catheter of Embodiment 1, and further description thereof is, therefore, deemed unnecessary. Differing from that of Embodiment 1, an inner tube 102 in the balloon catheter of Embodiment 2 includes an inner tube main segment 1021, a second recess 1022 and an inner-tube distal section 1023. The second recess 1022 is located at a distal end of the inner tube main segment 1021 and has an outer diameter smaller than an outer diameter of the inner tube main segment 1021. A proximal end of the balloon 200 is secured to a first recess 1012 and a distal end thereof is secured to the second recess 1022. The inner-tube distal section 1023 is disposed at a distal end of the second recess 1022 and has an outer diameter smaller than the outer diameter of the second recess 1022. The inner-tube distal section 1023 located at an end of the balloon catheter. The presence of the inner-tube distal section 1023 makes compliance of the catheter gradually increase from its proximal to distal end, ensuring satisfactory delivery performance and accessibility of the balloon catheter.

As shown in Fig. 4, the inner-tube distal section 1023 includes a proximal fourth transition section 1023-1 and a distal fourth straight section 1023-2. An outer diameter of the fourth transition section 1023-1 varies from an outer diameter of a third straight section 1022-2 to an outer diameter of the fourth straight section. The fourth transition section 1023-1 is a diameter-varying section where an outer diameter of the inner tube 102 decreases. In some embodiments, the outer diameter of the fourth straight section 1023-2 is 0.2 mm to 3.1 mm. In Embodiment 2, the outer diameter of the fourth straight section 1023-2 is 2.0 mm. In some other embodiments, the outer diameter of the fourth straight section 1023-2 may be 0.2 mm. In some other embodiments, the outer diameter of the fourth straight section 1023-2 may be 1.5 mm. In some other embodiments, the outer diameter of the fourth straight section 1023-2 may be 3.1 mm. In Embodiment 2, the outer diameter of the fourth straight section 1023-2 is smaller than the outer diameter of the second recess 1022, and an inner diameter of the fourth straight section 1023-2 is equal to an inner diameter of the second recess 1022. In some other embodiments, the outer diameter of the fourth straight section 1023-2 may be smaller than the outer diameter of the second recess 1022, and the inner diameter of the fourth straight section 1023-2 may be greater than the inner diameter of the second recess 1022. Through providing the fourth straight section 1023-2 at the distal end of the inner tube 102, the fourth straight section 1023-2 has a smaller outer diameter than that of the inner tube 102 at the proximal end thereof, the stiffness of the balloon catheter at the distal end thereof can be additionally reduced, enhancing the ability of the balloon catheter to pass through a blood vessel, reducing the risk of the distal end of the catheter causing damage to the blood vessel and improving its accessibility.

In some other embodiments, the inner-tube distal section 1023 may include 2-10 transition sections and straight sections. The transition sections may alternate with the straight sections, resulting in a gradually decreasing outer diameter profile of the inner-tube distal section 1023. The outer diameter of the inner-tube distal section 1023 may gradually decrease from 3 mm at the proximal end to 0.6 mm at the distal end. In some other embodiments, the inner-tube distal section 1023 may include 5 alternating transition and straight sections, and the outer diameter of the inner-tube distal section 1023 may decrease from 2.7 mm at the proximal end to 0.9 mm at the distal end. In some other embodiments, the inner-tube distal section 1023 may include 10 alternating transition and straight sections, and the outer diameter of the inner-tube distal section 1023 may decrease from 3.0 mm at the proximal end to 0.6 mm at the distal end. In some other embodiments, the inner-tube distal section 1023 may include 2 alternating transition and straight sections, and the outer diameter of the inner-tube distal section 1023 may decrease from 2.4 mm at the proximal end to 1.65 mm at the distal end. In some other embodiments, the inner-tube distal section 1023 may be a taped tubular structure with a gradually decreasing outer diameter. In some embodiments, the outer diameter of the inner-tube distal section 1023 may decrease from 3 mm at the proximal end to 0.6 mm at the distal end. In some embodiments, the outer diameter of the inner-tube distal section 1023 may decrease from 2.5 mm at the proximal end to 0.6 mm at the distal end. In some embodiments, the outer diameter of the inner-tube distal section 1023 may decrease from 2 mm at the proximal end to 0.9 mm at the distal end.

In Embodiment 2, a projection of a first transition location 300 on an axis of a tubular component 100 is distal with respect to a projection of a second transition location 400 on the axis of the tubular component 100. In some embodiments, the projection of the second transition location 400 on the axis of the tubular component 100 may be 10 mm to 80 mm away from the projection of the first transition location 300 on the axis of the tubular component 100. In Embodiment 2, the projection of the second transition location 400 on the axis of the tubular component 100 is 20 mm away from the projection of the first transition location 300 on the axis of the tubular component 100. In some other embodiments, the projection of the second transition location 400 on the axis of the tubular component 100 may be 10 mm away from the projection of the first transition location 300 on the axis of the tubular component 100. In some other embodiments, the projection of the second transition location 400 on the axis of the tubular component 100 may be 30 mm away from the projection of the first transition location 300 on the axis of the tubular component 100. In some other embodiments, the projection of the second transition location 400 on the axis of the tubular component 100 may be 40 mm away from the projection of the first transition location 300 on the axis of the tubular component 100. In some other embodiments, the projection of the second transition location 400 on the axis of the tubular component 100 may be 60 mm away from the projection of the first transition location 300 on the axis of the tubular component 100. In some other embodiments, the projection of the second transition location 400 on the axis of the tubular component 100 may be 80 mm away from the projection of the first transition location 300 on the axis of the tubular component 100. Through arranging the first transition location 300 on the distal side of the second transition location 400, it can be ensured that the size of the flow passage lumen will not shrink too much due to the reduced inner diameter of the outer tube 101, thus ensuring sufficient passage or suction efficiency. Further, through configuring the axial distance between the first transition location 300 and the second transition location 400 within a suitable range, in addition to a sufficient size of the flow passage lumen, good proximal supportability and distal flexibility of the balloon catheter can be ensuring, which enable the balloon catheter to have desirable ability to pass through bends and desirable accessibility.

In Embodiment 2, the inner tube 102 is a triple-layered structure consisting of an innermost first polymer layer, an immediate reinforcing layer and an outermost second polymer layer. The outer tube 101 is a double-layered structure consisting of an outer polymer layer and an inner reinforcing layer. The reinforcing layer of the outer tube 101 is provided by a cut tube.

In Embodiment 2, the balloon catheter includes a second radiopaque ring disposed at a location of the inner tube 102 that is adapted to the location of the balloon 200.

In Embodiment 2, there are angled transitions between the second recess 1022 and the inner-tube distal section 1023, and between the fourth transition section 1023-1 and the fourth straight section 1023-2. In some other embodiments, there may be smooth curved transition(s) between the second recess 1022 and the inner-tube distal section 1023 and/or between the fourth transition section 1023-1 and the fourth straight section 1023-2. In Embodiment 2, the fourth straight section 1023-2 has a straight, smooth surface. In some other embodiments, the fourth straight section 1023-2 may provide on its surface convexities and concavities, grooves or curved portions, while having constant inner and outer diameters across its entire length.

### Embodiment 3

In a third embodiment of the present invention, there is provided a balloon catheter. Fig. 5 is a cross-sectional view of a distal portion of this balloon catheter with a balloon 200 thereof being in an expanded configuration. As shown in Fig. 5, the balloon catheter of Embodiment 3 has an overall structure substantially similar to that of the balloon catheter of Embodiment 1, and further description thereof is, therefore, deemed unnecessary. Differing from that of Embodiment 1, in the balloon catheter of Embodiment 3, a first transition section 1012-1 in a first recess 1012 is a diameter-varying section perpendicular to an axis of a tubular component 100. That is, both outer and inner surfaces of the first transition section 1012-1 form a same angle of 90° with respect to the axis of the tubular component 100. An axial length of the first transition section 1012-1, i.e., a wall thickness of the tube there, is 0.1 mm, and an axial length of the first recess 1012 is 5 mm.

In Embodiment 3, a projection of a first transition location 300 on the axis of the tubular component 100 is distal with respect to a projection of a second transition location 400 on the axis of the tubular component 100.

In Embodiment 3, the inner tube 102 is a triple-layered structure consisting of an innermost first polymer layer, an immediate reinforcing layer and an outermost second polymer layer. The outer tube 101 is a double-layered structure consisting of an outer polymer layer and an inner reinforcing layer. The reinforcing layer of the outer tube 101 is provided by a cut tube.

In Embodiment 3, the balloon catheter includes a first radiopaque ring located at an end of the balloon catheter, and second and third radiopaque rings disposed at locations of the inner tube 102 that are adapted to the location of the balloon 200. Specifically, the second radiopaque ring may be disposed at a distal end of the balloon 200, and the third radiopaque ring may be disposed at a proximal end of the balloon 200.

### Embodiment 4

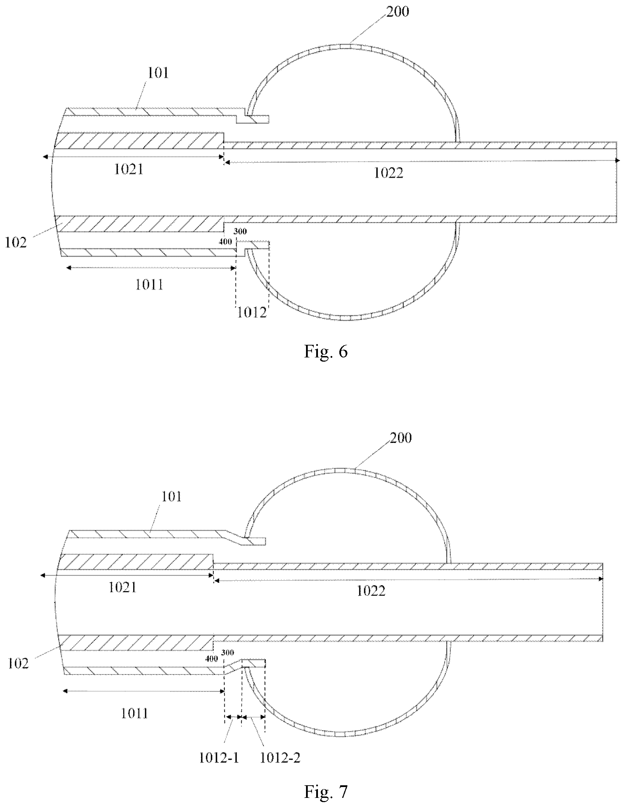

In a fourth embodiment of the present invention, there is provided a balloon catheter. Fig. 6 is a cross-sectional view of a distal portion of this balloon catheter with a balloon 200 thereof being in an expanded configuration. As shown in Fig. 6, the balloon catheter of Embodiment 4 has an overall structure substantially similar to that of the balloon catheter of Embodiment 3, and further description thereof is, therefore, deemed unnecessary. Differing from that of Embodiment 3, in the balloon catheter of Embodiment 4, a third transition section 1022-1 in a second recess 1012 is a diameter-varying section perpendicular to an axis of a tubular component 100. That is, both outer and inner surfaces of the third transition section 1022-1 form a same angle of 90° with respect to the axis of the tubular component 100. An axial length of a first transition section 1013-1 is 0.5 mm, and an axial length of a first recess 1012 is 8 mm. The first transition section 1012-1 in the first recess 1012 is a diameter-varying section perpendicular to the axis of the tubular component 100. That is, an outer surface of the first transition section 1012-1 forms an angle of 90° with respect to the axis of the tubular component 100. An axial length of the third transition section 1022-1 may be 0 mm, and an axial length of the second recess 1022 may be 20 mm. In Embodiment 4, an inner diameter of the second recess 1022 is equal to an inner diameter of an inner tube main segment 1021, and a thickness of the inner tube 102 at a distal end thereof is smaller than a thickness of the inner tube main segment 1021. In some other embodiments, an outer diameter of the second recess 1022 may be smaller than an outer diameter of the inner tube main segment 1021, the inner diameter of the second recess 1022 may be greater than the inner diameter of the inner tube main segment 1021, and the thickness of the inner tube at the distal end thereof may be smaller than the thickness of the inner tube main segment 1021. In some other embodiments, the outer diameter of the second recess 1022 may be smaller than the outer diameter of the inner tube main segment 1021, and the inner diameter of the second recess 1022 may be smaller than the inner diameter of the inner tube main segment 1021.

In Embodiment 4, a projection of a first transition location 300 on an axis of a tubular component 100 is distal with respect to a projection of a second transition location 400 on the axis of the tubular component 100. In some embodiments, the projection of the second transition location 400 on the axis of the tubular component 100 may be 10 mm to 80 mm away from the projection of the first transition location 300 on the axis of the tubular component 100. In Embodiment 4, the projection of the second transition location 400 on the axis of the tubular component 100 is 25 mm away from the projection of the first transition location 300 on the axis of the tubular component 100. In some other embodiments, the projection of the second transition location 400 on the axis of the tubular component 100 may be 15 mm away from the projection of the first transition location 300 on the axis of the tubular component 100. In some other embodiments, the projection of the second transition location 400 on the axis of the tubular component 100 may be 45 mm away from the projection of the first transition location 300 on the axis of the tubular component 100. In some other embodiments, the projection of the second transition location 400 on the axis of the tubular component 100 may be 65 mm away from the projection of the first transition location 300 on the axis of the tubular component 100. In some other embodiments, the projection of the second transition location 400 on the axis of the tubular component 100 may be 75 mm away from the projection of the first transition location 300 on the axis of the tubular component 100.

In Embodiment 4, the inner tube 102 is a double-layered structure consisting of an inner first polymer layer and an outer second polymer layer. The outer tube 101 is a single-layered polymer structure.

In Embodiment 4, the balloon catheter includes second and third radiopaque rings disposed at locations of the inner tube 102 that are adapted to the location of the balloon 200. Specifically, the second radiopaque ring may be disposed at a distal end of the balloon 200, and the third radiopaque ring may be disposed at a proximal end of the balloon 200.

As shown in Fig. 7, in some other embodiments, the third transition section 1022-1 in the second recess 1012 may be a diameter-varying section perpendicular to the axis of the tubular component 100. That is, both the outer and inner surfaces of the third transition section 1022-1 may form a same angle of 90° with respect to the axis of the tubular component 100. The axial length of the third transition section 1022-1 may be 0.05 mm, and the axial length of the second recess 1022 may be 8 mm. Additionally, both the inner and outer surfaces of the first transition section 1012-1 in the first recess 1012 may be inclined at angles with respect to the axis of the tubular component 100, which may be both 40°.

### Embodiment 5

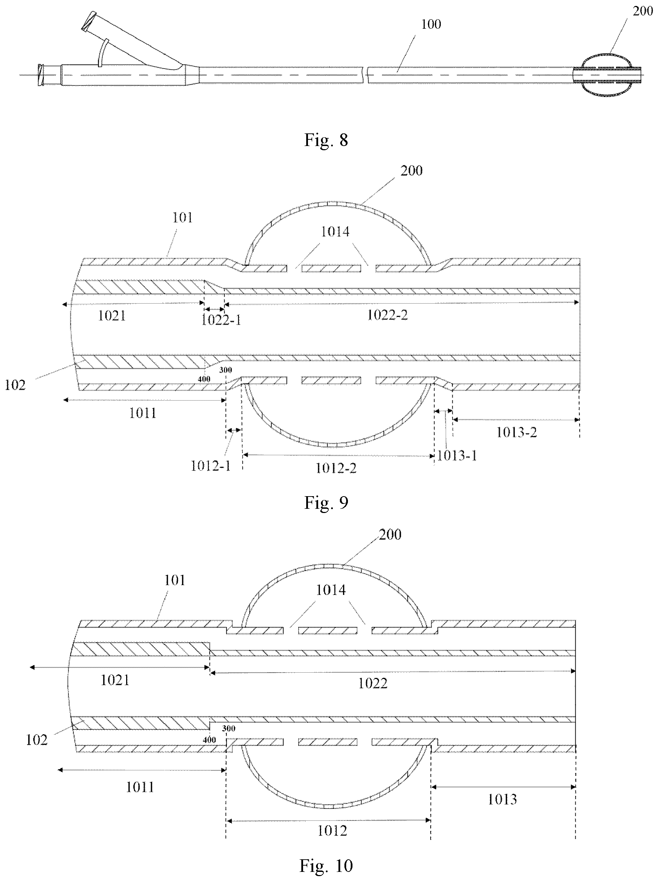

In a fifth embodiment of the present invention, there is provided a balloon catheter. Fig. 8 is a schematic overview of this balloon catheter, and Fig. 9 is a cross-sectional view of a distal portion thereof. As shown in Figs. 8 and 9, the balloon catheter of the present invention includes a tubular component 100 and a balloon 200. The tubular component 100 includes an outer tube 101 and an inner tube 102. The balloon 200 is secured to the outer tube 101 of the tubular component 100. The outer tube 101 is sleeved over the inner tube 102, and a first lumen is formed between the outer tube 101 and the inner tube 102. The balloon 200 has an expanded configuration and a contracted configuration. The balloon 200 is able to switch between the expanded and contracted configurations. As shown in Fig. 9, the outer tube 101 includes an outer tube main segment 1011 and a first recess 1012 located at a distal end of the outer tube main segment 1011. The balloon 200 is secured to the first recess 1012. Liquid passage apertures 1014 are formed in the first recess 1012. The first lumen is configured for passage or suction of a liquid therethrough, which can cause a switching of the balloon 200 between the expanded and contracted configurations. Filling the first lumen with a liquid can bring the balloon 200 into the expanded configuration, and emptying the first lumen can bring the balloon 200 into the contracted configuration. The liquid passage apertures 1014 are configured for passage of a liquid therethrough from the first lumen into the balloon 200 to bring the balloon 200 into the expanded configuration. The liquid may be sucked out from the balloon 200 to bring the balloon 200 into the contracted configuration. The first recess 1012 has been described above in detail in Embodiment 1 to 4 and, therefore, needs not be described in further detail here.

As shown in Fig. 9, in Embodiment 5, the outer tube 101 further includes an outer-tube distal section 1013 located at a distal end of the first recess 1012 and having an outer diameter at a proximal end thereof that is greater than an outer diameter of the first recess 1012 at the distal end thereof. The outer-tube distal section 1013 is fixedly coupled at a distal end thereof to the inner tube 102. The outer-tube distal section 1013 includes a second transition section 1013-1 and a second straight section 1013-2 from the proximal end to the distal end. An inner diameter of the second straight section 1013-2 is greater than an inner diameter of the first straight section 1012-2, and an outer diameter of the second straight section 1013-2 is greater than an outer diameter of the first straight section 1012-2. Inner and outer diameters of the second transition section 1013-1 gradually vary from the inner and outer diameters of the first straight section 1012-2 to the inner and outer diameters of the second straight section 1013-2, respectively. The second transition section 1013-1 is a diameter-varying section where inner and outer diameters of the outer tube 101 both increase. In some other embodiments, the inner diameter of the second straight section 1013-2 may be equal to the inner diameter of the first straight section 1012-2, and the outer diameter of the second straight section 1013-2 may be greater than the outer diameter of the first straight section 1012-2. Additionally, the outer diameter of the second transition section 1013-1 may gradually vary from the outer diameter of the proximal first straight section 1012-2 to the outer diameter of the distal second straight section 1013-2, with the inner diameter of the second transition section 1013-1 remaining constant. The second transition section 1013-1 is a diameter-varying section where the outer diameter of the outer tube 101 increases. The outer-tube distal section 1013 and the first recess may be connected to each other to form a V-shaped, frame-like, curved, polygonal, irregular or other recess. In Embodiment 5, the balloon 200 is connected at both proximal and distal end thereof to the first straight section 1012-2, optionally by gluing, bonding or fusion. In some other embodiments, a proximal end of the balloon 200 may be connected to the first transition section 1012-1 and/or a distal thereof may be connected to the second transition section 1013-1, optionally by gluing, bonding or fusion.

In Embodiment 5, inner and outer surfaces of the second transition section 1013-1 are inclined both at a same angle of 60° with respect to an axis of the tubular component 100. In some other embodiments, the inner and outer surfaces of the second transition section 1013-1 may be inclined both at the same or different angles with respect to the axis of the tubular component 100, which may each be any value in the range of 0-90°, such as 5°, 15°, 30°, 40°, 45°, 60°, 75° or 85°. In some other embodiments, the inner and outer surfaces of the second transition section 1013-1 may be both perpendicular to the axis of the tubular component 100. In some other embodiments, the inner surface of the second transition section 1013-1 may be parallel to the axis of the tubular component 100, and the outer surface of the second transition section 1013-1 may be included at an angle with respect to the axis of the tubular component 100, which may be any value in the range of 0-90°, such as 5°, 15°, 30°, 40°, 45°, 60°, 75° or 85°. In some other embodiments, the inner surface of the second transition section 1013-1 may be parallel to the axis of the tubular component 100, and the outer surface of the second transition section 1013-1 may be perpendicular to the axis of the tubular component 100. In each case, an axial length of the second transition section 1013-1 is 0-10 mm. In Embodiment 5, the axial length of the second transition section 1013-1 is 5 mm. In some other embodiments, the axial length of the second transition section 1013-1 may be 0 mm. In some other embodiments, the axial length of the second transition section 1013-1 may be 3 mm. In some other embodiments, the axial length of the second transition section 1013-1 may be 8 mm. In some other embodiments, the axial length of the second transition section 1013-1 may be 10 mm. In each case, an axial length of the outer-tube distal section 1013 is 1-15 mm. In Embodiment 5, the axial length of the outer-tube distal section 1013 is 10 mm. In some other embodiments, the axial length of the outer-tube distal section 1013 may be 1 mm. In some other embodiments, the axial length of the outer-tube distal section 1013 may be 8 mm. In some other embodiments, the axial length of the outer-tube distal section 1013 may be 12 mm. In some other embodiments, the axial length of the outer-tube distal section 1013 may be 15 mm.

In each case, the outer diameter of the second straight section 1013-2 is 1.0-3.7 mm. In Embodiment 5, the outer diameter of the second straight section 1013-2 is 2.8 mm. In some other embodiments, the outer diameter of the second straight section 1013-2 may be 1.0 mm. In some other embodiments, the outer diameter of the second straight section 1013-2 may be 2.0 mm. In some other embodiments, the outer diameter of the second straight section 1013-2 may be 3.0 mm. In some other embodiments, the outer diameter of the second straight section 1013-2 may be 3.7 mm.

In Embodiment 5, the outer-tube distal section 1013 is connected to the inner tube 102 (not shown in Fig. 9) so that the distal end of the first lumen is closed. As a result, when a liquid is flowing through the first lumen, it will not leak from the distal end of the catheter, thereby enabling expansion and contraction of the balloon 200. To this end, there may be a diameter-varying section (not shown) at the distal end of the outer-tube distal section 1013, which may have outer diameter gradually decreasing from its proximal to distal end and can be connected to the inner tube 102. The outer-tube distal section 1013 may be connected to the inner tube 102 either at the most distal end of the inner tube 102, or at another location of the inner tube 102.

As shown in Fig. 9, the inner tube 102 in the catheter of this embodiment may include an inner tube main segment 1021 and a second recess 1022 from the proximal end to the distal end. The second recess 1022 is located at a distal end of the inner tube main segment 1021 and has an outer diameter smaller than an outer diameter of the inner tube main segment 1021. The second recess 1022 has been described above in detail in Embodiment 1 to 4 and, therefore, needs not be described in further detail here. In some other embodiments, the inner tube 102 in the balloon catheter may include an inner tube main segment 1021, a second recess 1022 located at a distal end of the inner tube main segment 1021, and an inner-tube distal section 1023 located at a distal end of the second recess 1022. An outer diameter of the second recess 1022 is smaller than an outer diameter of the inner tube main segment 1021, and an outer diameter of the inner-tube distal section 1023 is smaller than the outer diameter of the second recess 1022.

In Embodiment 5, a projection of a first transition location 300 on an axis of a tubular component 100 is distal with respect to a projection of a second transition location 400 on the axis of the tubular component 100. In some embodiments, the projection of the second transition location 400 on the axis of the tubular component 100 may be 10 mm to 80 mm away from the projection of the first transition location 300 on the axis of the tubular component 100. In Embodiment 5, the projection of the second transition location 400 on the axis of the tubular component 100 is 18 mm away from the projection of the first transition location 300 on the axis of the tubular component 100. In some other embodiments, the projection of the second transition location 400 on the axis of the tubular component 100 may be 12 mm away from the projection of the first transition location 300 on the axis of the tubular component 100. In some other embodiments, the projection of the second transition location 400 on the axis of the tubular component 100 may be 42 mm away from the projection of the first transition location 300 on the axis of the tubular component 100. In some other embodiments, the projection of the second transition location 400 on the axis of the tubular component 100 may be 55 mm away from the projection of the first transition location 300 on the axis of the tubular component 100. In some other embodiments, the projection of the second transition location 400 on the axis of the tubular component 100 may be 78 mm away from the projection of the first transition location 300 on the axis of the tubular component 100.

In Embodiment 5, the inner tube 102 is a triple-layered structure consisting of an innermost first polymer layer, an immediate reinforcing layer and an outermost second polymer layer. The outer tube 101 is a double-layered structure consisting of an outer polymer layer and an inner polymer layer.

In Embodiment 5, the catheter includes a first radiopaque ring sleeved over the inner tube and located at an end of the balloon catheter. The catheter also includes a second radiopaque ring disposed at a location of the inner tube 102 that is adapted to the location of the balloon 200.

As shown in Fig. 10, in some other embodiments, in the outer-tube distal section 1013, the second transition section 1013-1 may be a diameter-varying section perpendicular to the axis of the tubular component 100. That is, the outer and inner surfaces of the second transition section 1013-1 may both form a same angle of 90° with respect to the axis of the tubular component 100.

In Embodiment 5, there are angled transitions between the first recess 1012 and the outer-tube distal section 1013 and between the second transition section 1013-1 and the second straight section 1013-2. In some other embodiments, there may be smooth curved transition(s) between the first recess 1012 and the outer-tube distal section 1013 and/or between the second transition section 1013-1 and the second straight section 1013-2. In Embodiment 5, the second straight section 1013-2 has a straight, smooth surface. In some other embodiments, the second straight section 1013-2 may provide on its surface with convexities and concavities, grooves or curved portions, while having constant inner and outer diameters across its entire length.

The foregoing description presents some preferred embodiments of the present invention and is not intended to limit the scope of the present invention in any way. Any and all changes and modifications made by those of ordinary skill in the art in light of the above teachings without departing from the spirit of the present invention are intended to be embraced in the scope as defined by the appended claims.

## Claims

1. A balloon catheter, comprising a tubular component and a balloon, wherein: the tubular component comprises an inner tube and an outer tube; the balloon is secured to the tubular component, and has an expanded configuration and a contracted configuration; the outer tube is sleeved over the inner tube; and a first lumen is formed between the outer tube and the inner tube,
wherein the outer tube comprises an outer tube main segment and a first recess, wherein: the first recess is located at a distal end of the outer tube main segment; the first recess has an outer diameter smaller than an outer diameter of the outer tube main segment; and the first recess has an inner diameter smaller than an inner diameter of the outer tube main segment, and wherein a proximal end of the balloon is fixedly connected to the first recess, and
wherein the inner tube comprises an inner tube main segment and a second recess, wherein the second recess is located at a distal end of the inner tube main segment, and wherein the second recess has an outer diameter smaller than an outer diameter of the inner tube main segment.

2. The balloon catheter of claim 1, wherein the first recess comprises a first transition section and a first straight section from a proximal end to a distal end thereof, wherein the first transition section is a diameter-varying section where an inner diameter and an outer diameter of the outer tube decrease.

3. The balloon catheter of claim 2, wherein the first transition section has an axial length of 0 mm to 10 mm.

4. The balloon catheter of claim 2, wherein an inner surface and an outer surface of the first transition section are inclined at a same angle of 0°-90° with respect to an axis of the outer tube main segment.

5. The balloon catheter of claim 2, wherein a ratio of an outer diameter of the first straight section to the outer diameter of the outer tube main segment is 0.7-1.0.

6. The balloon catheter of claim 5, wherein the outer diameter of the outer tube main segment is 1.0 mm to 3.7 mm, and wherein the outer diameter of the first straight section is 0.7 mm to 3.5 mm.

7. The balloon catheter of claim 1, wherein the outer tube further comprises an outer-tube distal section located at a distal end of the first recess, wherein an outer diameter of the outer-tube distal section at a proximal end thereof is greater than the outer diameter of the first recess at the distal end thereof, and wherein the outer-tube distal section is fixedly connected to the inner tube at a distal location.

8. The balloon catheter of claim 7, wherein the outer-tube distal section comprises a second transition section and a second straight section from a proximal end to a distal end thereof, and wherein the second transition section is a diameter-varying section where an outer diameter of the outer tube increases.

9. The balloon catheter of claim 1, wherein the second recess comprises a third transition section and a third straight section from a proximal end to a distal end thereof, and wherein the third transition section is a diameter-varying section where an outer diameter of the inner tube decreases.

10. The balloon catheter of claim 9, wherein the second recess has an axial length of 2-60 mm.

11. The balloon catheter of claim 9, wherein an outer surface of the third transition section is inclined at an angle of 0°-90° with respect to an axis of the inner tube main segment, and wherein the third transition section has an axial length of 0-10mm.

12. The balloon catheter of claim 9, wherein a ratio of an outer diameter of the third straight section to the outer diameter of the inner tube main segment is greater than or equal to 0.6 and is smaller than 1.0.

13. The balloon catheter of claim 12, wherein the outer diameter of the inner tube main segment is 0.5 mm to 3.2 mm, and wherein the outer diameter of the third straight section is greater than or equal to 0.3 mm and is smaller than 3.2 mm.

14. The balloon catheter of claim 1, wherein the inner tube further comprises an inner-tube distal section located at a distal end of the second recess.

15. The balloon catheter of claim 14, wherein the inner-tube distal section has an axial length of 1-500 mm.

16. The balloon catheter of claim 14, wherein an outer diameter of the inner-tube distal section is smaller than the outer diameter of the second recess, and wherein the inner-tube distal section is located at a distal end of the balloon catheter.

17. The balloon catheter of claim 14, wherein the inner-tube distal section comprises a fourth transition section and a fourth straight section from a proximal end to a distal end thereof, and wherein the fourth transition section is a diameter-varying section where an outer diameter of the inner tube decreases.

18. The balloon catheter of claim 17, wherein the fourth straight section has an outer diameter of 0.2 mm to 3.1 mm.

19. The balloon catheter of claim 1, wherein a most distal end of the outer tube main segment defines a first transition location, wherein a most distal end of the inner tube main segment defines a second transition location, and wherein the second transition location is located proximally with respect to the first transition location.

20. The balloon catheter of claim 19, wherein the first recess comprises a first transition section and a first straight section from a proximal end to a distal end thereof, and wherein the second recess comprises a third transition section and a third straight section from a proximal end to a distal end thereof,
wherein: an outer surface of the third transition section forms a first angle with respect to an axis of the tubular component; an inner surface of the first transition section forms a second angle with respect to the axis of the tubular component; and the first angle is greater than or equal to the second angle.

21. The balloon catheter of claim 20, wherein a projection of the second transition location on the axis of the tubular component is 10 mm to 80 mm away from a projection of the first transition location on the axis of the tubular component.

22. The balloon catheter of claim 21, wherein the projection of the second transition location on the axis of the tubular component is 20 mm to 60 mm away from the projection of the first transition location on the axis of the tubular component.

23. The balloon catheter of claim 22, wherein the projection of the second transition location on the axis of the tubular component is 30 mm to 45 mm away from the projection of the first transition location on the axis of the tubular component.

24. The balloon catheter of claim 1, wherein a distal end of the balloon is fixedly connected to the second recess.

25. The balloon catheter of claim 1, wherein the balloon is disposed on the first recess, wherein each of a proximal end and a distal end of the balloon is fixedly connected to the outer tube, wherein a distal end of the outer tube is connected to the inner tube, and wherein the first recess is provided with liquid passage apertures for dilation of the balloon with a liquid.

26. The balloon catheter of claim 1, wherein the balloon is made of any one of silicone, polyurethane, latex, polyethylene, polytrafluoroethylene and expanded polytrafluoroethylene, or of a mixture thereof.

27. The balloon catheter of claim 1, wherein each of the inner and outer tubes comprises at least one polymer layer, and wherein the polymer layer is made of one or more of polyether block amide, nylon, polyurethane, polytrafluoroethylene, polyethylene and polyolefin elastomer.

28. The balloon catheter of claim 27, wherein the outer tube and/or the inner tube further comprise(s) reinforcing layer(s), wherein the reinforcing layer is a structure braided from filaments, a structure consisting of spirally wound filaments, a cut tube or a combination thereof, and wherein the reinforcing layer is made of stainless steel, a nickel-titanium alloy, a cobalt-chromium alloy or a polymer.

29. The balloon catheter of claim 27, wherein the outer tube and/or the inner tube is/are triple-layered structure(s) each consisting of an innermost first polymer layer, an intermediate reinforcing layer and an outermost second polymer layer.

30. The balloon catheter of claim 1, wherein the inner tube provides therein with a second lumen, wherein the second lumen has a same inner diameter across an entire length thereof.

31. The balloon catheter of claim 30, wherein a ratio of the inner diameter of the second lumen to the outer diameter of the outer tube main segment is 0.2-0.9.

32. The balloon catheter of claim 31, wherein the inner diameter of the second lumen is 0.1 mm to 3.0 mm, and wherein the outer diameter of the outer tube main segment is 0.5 mm to 3.7 mm.

33. The balloon catheter of claim 1, wherein the balloon has a length of 5-30 mm in the contracted configuration.

34. The balloon catheter of claim 33, wherein the balloon has a length of 10-20 mm in the contracted configuration.
